# EUROPÄISCHE PATENTSCHRIFT

(11) **EP 3 873 888 B1**
(45) Veröffentlichungstag und Bekanntmachung des Hinweises auf die Patenterteilung: **18.03.2026**
(21) Anmeldenummer: 19794999.3
(22) Anmeldetag: 28.10.2019
(51) Int. Cl.: C07D 309/04

(54) **HERSTELLUNG VON 2-SUBSTITUIERTEN 4-METHYL-TETRAHYDROPYRANEN AUS 2-SUBSTITUIERTEN 4-HYDROXY-4-METHYL-TETRAHYDROPYRANEN ALS AUSGANGSSTOFFEN**
PREPARATION OF 2-SUBSTITUTED 4-METHYL-TETRAHYDROPYRANS OF 2-SUBSTITUTED 4 HYDROXY-4-METHYL-TETRAHYDROPYRANS AS STARTING MATERIALS
PRODUCTION DE 4-MÉTHYLE-TÉTRAHYDROPYRANES 2-SUBSTITUÉS À PARTIR DE 4-HYDROXY-4 MÉTHYLE-TÉTRAHYDROPYRANES 2-SUBSTITUÉS EN TANT QUE MATIÈRES PREMIÈRES

(30) Priorität: 29.10.2018 EP 18203122
(43) Veröffentlichungstag der Anmeldung: 08.09.2021
(73) Patentinhaber: BASF SE, 67056 Ludwigshafen am Rhein (DE)
(72) Erfinder: STOCK, Christoph, 67056 Ludwigshafen (DE); DE WISPELAERE, Irene, 2040 Antwerpen (NL); BRUNNER, Bernhard, 67056 Ludwigshafen (DE); KRAUSE, Wolfgang, 68623 Lampertheim (DE); GARLICHS, Florian, 68623 Lampertheim (DE)
(74) Vertreter: BASF IP Association
(86) Internationale Anmeldenummer: PCT/EP2019/079322
(87) Internationale Veröffentlichungsnummer: WO 2020/089134

(56) Entgegenhaltungen:
- EP-A1- 2 112 144
- US-A1- 2009 263 336

## Beschreibung

### HINTERGRUND DER ERFINDUNG

Die vorliegende Erfindung betrifft ein Verfahren zur Herstellung von 2-substituierten 4-Methyl-tetrahydropyranen aus 2-substituierten 4-Hydroxy-4-methyl-tetrahydropyranen als Ausgangsstoffen.

### STAND DER TECHNIK

Alkylsubstituierte Tetrahydropyrane haben weite Verbreitung als Aroma- und Geschmacksstoffe gefunden. Ein bekannter Vertreter aus dieser Klasse ist das 2-Isobutyl-4-methyl-tetrahydropyran (Dihydrorosenoxid oder Dihydrorosan ^{®}), welches eine florale, grüne Duftnote aufweist.

Die Erstsynthese von Dihydrorosenoxid wurde von M. Julia und B. Jacquet Bulletin de la Societe Chimique de France 1963, 8-9, 1983, beschrieben. Ausgehend von But-2-en-1-al wurde durch eine Diels-Alder-Reaktion mit Ethylvinylether und anschließender Hydrierung ein cyclisches Acetal erhalten. Nach Abspaltung von Ethanol, Hydrobromierung der erhaltenen Doppelbindung und abschließender Grignard-Reaktion mit Isopropylmagnesiumbromid konnte eine racemische Mischung aus cis- und trans-Dihydrorosenoxid erhalten werden.

Liu et al. beschreiben in J. Heterocyclic Chem., 21, 129-132 (1984) die Herstellung von cis-Dihydrorosenoxid durch Hydrierung von 2-Isobutyl-4-methyl-5,6-dihydro-4H-pyran mit PtO₂ in Essigsäure.

Schindler und Vogel beschreiben in Perfume & Flavorist, Vol. 11, 29-30 (1986), schematisch die Herstellung von Dihydrorosenoxid aus 3-Methylbut-3-en-1-ol und 3-Methylbutanal als Ausgangsmaterial, wobei eine cis/trans-Mischung im Verhältnis 70 : 30 erhalten wird. Weder der Reaktionsweg noch die einzuhaltenden Bedingungen sind genauer beschrieben.

Die EP 0 770 670 B1 beschreibt eine Parfümzusammensetzung, die 2-substituierte (4R)-cis-4-Methyl-tetrahydro-2H-pyrane enthält. In der Anmeldung werden die Geruchseigenschaften der Isomeren von Rosenoxid und Dihydrorosenoxid beschrieben. Die Synthese der Isomeren des Dihydrorosenoxids erfolgt durch Hydrierung der entsprechenden Isomeren des Rosenoxids.

WO 2014/060345 beschreibt ein Verfahren zur Herstellung von 2-substituierten 4-Hydroxy-4-methyl-tetrahydropyranen und 2-substituierten 4-Methyl-tetrahydropyranen durch Umsetzung von Isoprenol (3-Methylbut-3-en-ol) mit einem Aldehyd. Im ersten Schritt wird Isoprenol in Gegenwart eines geeigneten Aldehyds umgesetzt, wobei ein Gemisch aus 2-substituierten 4-Hydroxy-4-methyl-tetrahydropyranen, 6-substituierten 4-Methyl-3,6-dihydro-2H-pyranen, 2-substituierte 4-Methylen-tetrahydropyranen, 2-substituierte 4-Methyl-3,6-dihydro-2H-pyranen und 2-substituierte 4,4-Dimethyl-1,3-dioxanen erhalten wird. Die Alkoholverbindung wird abgetrennt. Die verbleibenden Verbindungen werden einer Hydrierung unter Erhalt von 2-substituierten 4-Methyl-tetrahydropyranen und Dioxanverbindungen unterzogen.

WO 2015/158584 beschreibt ein Verfahren zur Herstellung von 2-substituierten 4-Hydroxy-4-methyl-tetrahydropyranen. 2-substituierte 4,4-Dimethyl-1,3-dioxane werden mit einer starken Säure umgesetzt, wobei ein Produktgemisch von 6-substituierten 4-Methyl-3,6-dihydro-2H-pyranen, 2-substituierten 4-Methylene-tetrahydropyranen und 2-substituierten 4-Methyl-3,6-dihydro-2H-pyranen erhalten wird. Das Produktgemisch wird einer Hydrierung unterzogen.

Sowohl US 2009/0263336 als auch EP 2 112 144 beschreiben die Herstellung von 2-Alkyl-4-methyl-tetrahydropyranol-Verbindungen. Das erhaltene Pyranol kann in einem weiteren Schritt durch Dehydrierung zu einem Gemisch aus 4-Methylen-2-alkyl-tetrahydropyran, 4-Methyl-2-alkyl-5,6-dihydropyran und 4-Methyl-2-alkyl-3,6-dihydropyran umgesetzt werden. Optional kann das erhaltene Gemisch hydriert werden, so dass die entsprechenden 4-Methyl-2-alkyl-tetrahydropyrane erhalten werden. Zum einen handelte es sich bei den Verfahren dieser Dokumente nicht um eine Eintopfsynthese. Jede Verbindung der Zwischenstufe muss für den darauffolgenden Schritt isoliert werden. Zum anderen erfolgt die Herstellung der 2-Alkyl-4-methyl-tetrahydropyran-Derivate in den genannten Dokumenten aus einem Gemisch der oben genannten Verbindungen durch Hydrierung. Ein Säurekatalysator ist nicht erwähnt. Die erwähnte Säure in diesen Dokumenten findet lediglich Einsatz bei der Herstellung des Pyranols.

Es besteht weiterhin ein großer Bedarf an effektiven Verfahren zur Herstellung von 2-substituierten 4-Methyl-tetrahydropyranen aus leicht verfügbaren Ausgangsstoffen. Neben der Synthese aus Reinsubstanzen ist dabei speziell auch die Nutzung von bisher nicht verwertbaren Nebenprodukten aus anderen Syntheseverfahren von Interesse.

Der vorliegenden Erfindung liegt die Aufgabe zugrunde, ein verbessertes Verfahren zur Herstellung von 2-substituierten 4-Methyl-tetrahydropyranen zur Verfügung zu stellen.

Überraschenderweise wurde jetzt gefunden, dass durch Hydrierung von 2-substituierten 4-Hydroxy-4-methyl-tetrahydropyranen in Gegenwart eines Hydrierkatalysators unter sauren Bedingungen auf schnellem Weg 2-substituierte 4-Methyl-tetrahydropyrane, speziell in Dihydrorosenoxid überführt werden kann.

### ZUSAMMENFASSUNG DER ERFINDUNG

Gegenstand der Erfindung ist ein Verfahren zur Herstellung von Verbindungen der allgemeinen Formel (I) 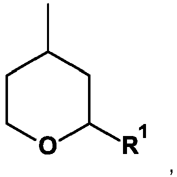 wobei R¹ ausgewählt ist unter
geradkettigem oder verzweigtem C₁-C₁₂-Alkyl, wobei Alkyl unsubstituiert oder mit wenigstens einen Substituenten, ausgewählt unter Aryl, C₁-C₁₂-Alkoxy und C₁-C₁₂-alkylcarbonyl aufweist,
unsubstituiertem oder mit 1, 2, 3 oder 4 Substituenten, ausgewählt unter C₁-C₁₂-Alkyl, C₁-C₁₂-Alkoxy, C₁-C₁₂-Alkyl, C₁-C₁₂-Alkoxy, Phenyl und
Benzyl, substituiertem Cycloalkyl mit insgesamt 3 bis 20 Kohlenstoffatomen,
umfassend die Schritte:
   a) Bereitstellung wenigstens einer Verbindung der allgemeinen Formel (II) 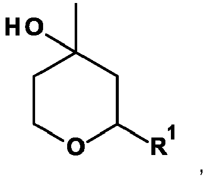 wobei R¹ die zuvor angegebene Bedeutung hat,
   b) Hydrierung der Verbindung (II) in Gegenwart eines Hydrierkatalysators unter sauren Bedingungen,
   wobei es sich um eine Eintopfsynthese handelt.

Ein weiterer Gegenstand der Erfindung ist ein Verfahren zur Herstellung von Verbindungen der allgemeinen Formel (I) 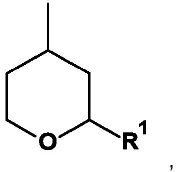 wobei R¹ ausgewählt ist unter
geradkettigem oder verzweigtem C₁-C₁₂-Alkyl, wobei Alkyl unsubstituiert oder mit wenigstens einen Substituenten, ausgewählt unter Aryl, C₁-C₁₂-Alkoxy und C₁-C₁₂-alkylcarbonyl aufweist,
unsubstituiertem oder mit 1, 2, 3 oder 4 Substituenten, ausgewählt unter C₁-C₁₂-Alkyl, C₁-C₁₂-Alkoxy, C₁-C₁₂-Alkyl, C₁-C₁₂-Alkoxy, Phenyl und
Benzyl, substituiertem Cycloalkyl mit insgesamt 3 bis 20 Kohlenstoffatomen,
umfassend die Schritte:
   a) Bereitstellung wenigstens einer Verbindung der allgemeinen Formel (II) 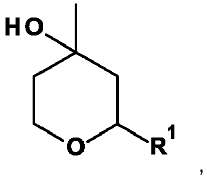 wobei R¹ die zuvor angegebene Bedeutung hat,
   b) Hydrierung der Verbindung (II) in Gegenwart eines Hydrierkatalysators unter sauren Bedingungen.

### BESCHREIBUNG DER ERFINDUNG

Das erfindungsgemäße Verfahren weist die folgenden Vorteile auf:
- Mit der erfindungsgemäß vorgesehenen Reaktion wird ein Zugang zu 2-substituierten 4-Methyl-tetrahydropyranen und speziell zu **Dihydrorosenoxid/Dihydrorosan** ^{®} ermöglicht, der nur eine Reaktionsstufe erfordert (Eintopfsynthese).
- Zur Herstellung der 2-substituierten 4-Methyl-tetrahydropyrane, speziell des Dihydrorosenoxids, müssen keine weiteren teuren und/oder potentiell gefährlichen Reagenzien, wie etwa Grignard-Reagenzien oder komplexe Hydride, wie Lithiumaluminiumhydrid, eingesetzt werden.

Eintopfsynthese im Sinne der Erfindung beschreibt eine Synthese, die nur eine Reaktionsstufe erfordert. Eine Isolierung von Zwischenverbindungen findet nicht statt. Die erfindungsgemäße Reaktion findet in situ statt. Mit anderen Worte alle für das erfindungsgemäße Verfahren benötigten Stoffe in den Schritten a) und b) sind bereits von Beginn an im Reaktionsgefäß vorhanden oder werden im Verlauf der Reaktion zugegeben, ohne jedoch die Reaktion zu stoppen. Sobald die Reaktion beendet ist, wird das gewünschte Produkt erhalten. Das Produkt kann gegebenenfalls gereinigt werden nach den üblichen, dem Fachmann bekannten Reinigungsverfahren, wie z. B. Filtration, Destillation, Extraktion oder eine Kombination davon.

### Sofern im Folgenden nicht genauer angegeben, bezeichnen die Begriffe

"2-substituiertes 4-Methyl-tetrahydropyran", "2-(2-Methylpropyl)-4-methyl-tetrahydropyran", "2-Isobutyl-4-methyl-tetrahydropyran" (= "Dihydrorosenoxid" oder "Dihydrorosan ^{®}"), "2-substituiertes 4-Hydroxy-4-methyl-tetrahydropyran", "2-(2-Methylpropyl)-4-hydroxy-4-methyl-tetrahydropyran", "2-Isobutyl-4-methyl-tetrahydropyran-4-ol" im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Sofern im Folgenden von cis- und trans-Diastereomeren der Verbindungen (I) oder (II) die Rede ist, wird jeweils nur eine der enantiomeren Formen abgebildet. Lediglich zur Veranschaulichung werden im Folgenden die Isomeren des 2-(2-Methylpropyl)-4-methyl-tetrahydropyrans (I) (Dihydrorosenoxid/Dihydrorosan ^{®}) wiedergegeben:

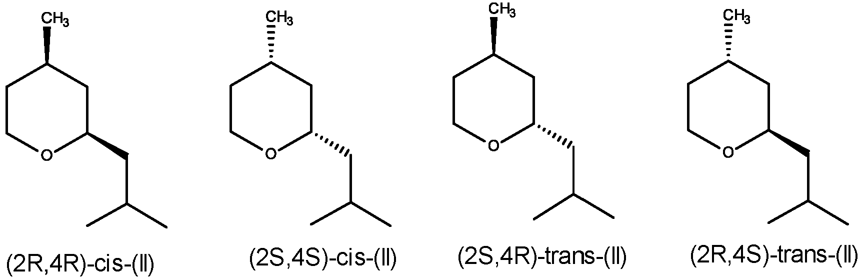

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl oder Isobutyl.

Im Rahmen der vorliegenden Erfindung steht der Ausdruck mit Aryl substituiertes Alkyl bevorzugt für mit Aryl substituiertes C₁-C₆-Alkyl und besonders bevorzugt für mit Aryl substituiertes C₁-C₄-Alkyl. Mit Aryl substituiertes Alkyl steht insbesondere für Benzyl, 1-Phenethyl oder 2-Phenethyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy oder Isobutyloxy.

Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl, C₁-C₆-Alkoxy, Phenyl und Benzyl, besonders bevorzugt C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-Isopropylcyclohexyl, 2-, 3- und 4-Butylcyclohexyl und 2-, 3- und 4-Isobutylcyclohexyl.

Im Rahmen der Erfindung steht der Ausdruck Alkyl-Carbonyl bevorzugt für (C₁-C₆-Alkyl)carbonyl, wobei Alkyl, wie oben definiert, über eine Carbonylgruppe an den Rest des Moleküls gebunden ist.

Der Ausdruck "Aryl" umfasst im Rahmen der vorliegenden Erfindung ein- oder mehrkernige aromatische Kohlenwasserstoffreste mit üblicherweise 6 bis 18, vorzugsweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc. und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl, 2,4,6-Tri-tert.-butylphenyl, 1-Methyl-2-naphthyl, 3-Methyl-2-naphthyl, 1,3-Dimethyl-2-naphthyl, 5,6,7,8-Tetramethyl-2-naphthyl, 5-Methyl-2-naphthyl, 6-Methyl-2-naphthyl, 7-Methyl-2-naphthyl, 8-Methyl-2-naphthyl.

Bevorzugt steht R¹ in den Verbindungen der Formel (I) und (II), für geradkettiges oder verzweigtes C₁-C₁₂-Alkyl, wobei Alkyl unsubstituiert oder mit Aryl substituiert ist. Besonders bevorzugt steht R¹ für geradkettiges oder verzweigtes C₁-C₆-Alkyl, wobei Alkyl unsubstituiert oder wenigstens einen Substituenten aufweist, der ausgewählt ist unter Phenyl und C₁-C₆-Alkoxy.

Erfindungsgemäß bevorzugte Bedeutungen für den Rest R¹ sind somit beispielsweise Methyl, Ethyl, n-Propyl, Isopropyl, n-Butyl, Isobutyl, n-Pentyl, n-Hexyl oder n-Heptyl, bevorzugt Methyl, Ethyl, n-Propyl, Isopropyl, n-Butyl, Isobutyl, ganz besonders bevorzugt Isobutyl (2-Methylpropyl).

Die vorliegende Erfindung betrifft somit im Rahmen einer bevorzugten Ausführungsform ein Verfahren zur Herstellung und Isolierung von 2-(2-Methylpropyl)-4-methyl-tetrahydropyran der Formel (la) (Dihydrorosenoxid/Dihydrorosan ^{®}).

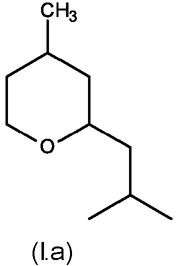

### Schritt a)

Geeignete Ausgangsmaterialien für den Einsatz in Schritt a) können wenigstens eine Verbindung der Formel (II) 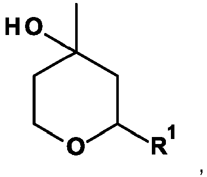 sein, wobei R¹ ausgewählt ist unter
geradkettigem oder verzweigtem C₁-C₁₂-Alkyl, wobei Alkyl unsubstituiert oder mit wenigstens einen Substituenten, ausgewählt unter Aryl, C₁-C₁₂-Alkoxy und C₁-C₁₂-alkylcarbonyl aufweist,
unsubstituiertem oder mit 1, 2, 3 oder 4 Substituenten, ausgewählt unter C₁-C₁₂-Alkyl, C₁-C₁₂-Alkoxy, C₁-C₁₂-Alkyl, C₁-C₁₂-Alkoxy, Phenyl und Benzyl, substituiertem Cycloalkyl mit insgesamt 3 bis 20 Kohlenstoffatomen.

Bevorzugt ist R¹ ausgewählt unter einem geradkettigem oder verzweigtem C₁-C₆-Alkyl, wobei Alkyl unsubstituiert oder wenigstens einen Substituenten aufweist, der ausgewählt ist unter Phenyl und C₁-C₆-Alkoxy.

Besonders bevorzugt ist R¹ ausgewählt unter Methyl, Ethyl, n-Propyl, Isopropyl, n-Butyl, Isobutyl, n-Pentyl, n-Hexyl und Phenyl.

Ganz besonders bevorzugt ist R¹ ausgewählt unter Methyl, Ethyl, n-Propyl, Isopropyl, n-Butyl, Isobutyl, n-Pentyl und n-Hexyl.

In einer speziellen Ausführungsform steht R¹ für Isobutyl (2-Methylpropyl).

Der Syntheseweg zur Herstellung der Verbindung der Formel (II) ist in WO 2010/133473, WO 2015/158454 und WO 2014/060345 beschrieben.

### Schritt b)

Erfindungsgemäß wird die Verbindung der Formel (II) einer Eliminierung gefolgt von einer Hydrierung in Gegenwart eines Hydrierkatalysators unter sauren Bedingungen unterzogen. Durch die Eliminierung und Hydrierung im Schritt b) wird die Verbindung der Formel (II) zu der entsprechenden Verbindung der Formel (I) umgewandelt.

Die Eliminierung gefolgt von einer Hydrierung erfolgt vorzugsweise in einer Reaktionsstufe (Eintopfsynthese), d. h. ohne Isolierung von Zwischenverbindungen.

Unter dem Bergiff "unter sauren Bedingungen" wird im Rahmen der Erfindung verstanden, dass die Reaktion in Gegenwart einer Säure stattfindet. Als Säure wird jede Substanz verstanden, die Brönsted-oder Lewis-Acidität aufweist.

Vorzugsweise sind solche Substanzen ausgewählt unter Protonendonatoren, Elektronenakzeptoren und Gemischen davon.

Protonendonatoren sind vorzugsweise ausgewählt unter molekularen Protonensäuren, Ionenaustauschern und Gemischen davon.

Elektronenakzeptoren sind vorzugsweise ausgewählt unter sauren molekularen Elementverbindungen, oxidischen sauren Festkörpern und Gemischen davon.

Geeignete molekulare Protonensäuren sind beispielsweise Salzsäure, Schwefelsäure, Phosphorsäure, Salpetersäure, Essigsäure, Ameisensäure, Trifluormethylsulfonsäure, Methansulfonsäure, p-Toluolsulfonsäure und Gemische davon.

Geigenete saure molekulare Elementverbindungen sind beispielsweise Aluminiumchlorid, Bortrifluorid, Zinkchlorid, Phosphorpentafluorid, Arsentrifluorid, Zinntetrachlorid, Titantetrachlorid, Antimonpentafluorid und Gemische davon.

Geigenete oxidische saure Festkörper sind beispielsweise Zeolithe, Silikate, Aluminate, Alumosilikate, Tone und Gemische davon.

### Geigenete Ionenaustauscher sind saure kationische Ionenaustauscher.

Unter dem Begriff "saurer Kationenaustauscher" sind dabei im Rahmen der vorliegenden Erfindung solche Kationenaustauscher in der H⁺-Form zu verstehen, die saure Gruppen, in der Regel Sulfonsäuregruppen, aufweisen, deren Matrix gelförmig bzw. makroporös sein kann. Eine bevorzugte Ausführungsform des erfindungsgemäßen Verfahrens ist dementsprechend dadurch gekennzeichnet, dass man einen sauren, Sulfonsäuregruppen aufweisenden bzw. umfassenden Kationenaustauscher einsetzt.

Saure Kationenaustauscher sind insbesondere lonenaustauscherharze in der H⁺-Form. Als solche kommen beispielsweise in Betracht:
- saure Ionenaustauscher (wie z. B. Amberlyst, Amberlite, Dowex, Lewatit, Purolite, Serdolit), die auf Polystyrol basieren, und die Copolymere aus Styrol und Divinylbenzol als Trägermatrix mit Sulfonsäuregruppen in H⁺-Form enthalten,
- mit Sulfonsäuregruppen (-SO₃H) funktionalisierte lonenaustauschergruppen.

Die Ionenaustauscher unterscheiden sich im Aufbau ihrer Polymergerüste, und man unterscheidet gelförmige und makroporöse Harze. Die sauren lonentauscherharze werden in der Regel mit Salzsäure und/oder Schwefelsäure regeneriert.

Nafion^{®} ist die Bezeichnung der Firma Dupont für perfluorierte polymere lonenaustauscherharze. Es handelt sich dabei um perfluorierte lonenaustauschmaterialien, bestehend aus Fluorkohlenstoff-Basisketten und perfluorierten Seitenketten, die Sulfonsäuregruppen enthalten. Die Harze werden durch eine Copolymerisation von perfluorierten, endständig ungesättigten und mit Sulfonylfluorid funktionalisierten Ethoxylaten mit Perfluorethen hergestellt. Nafion^{®} zählt zu den gelartigen lonenaustauscherharzen. Als Beispiel für ein solches perfluoriertes polymeres lonenaustauscherharz sei Nafion^{®}> NR-50 genannt.

Die sauren Kationenaustauscher werden in der Regel in der H⁺-Form einsetzt, wobei der Ionenaustauscher ein Sulfonsäuregruppen aufweisendes Polymergerüst enthält und entweder gelförmig ist oder makroporöse Harze enthält.

Eine ganz besonders bevorzugte Ausführungsform des erfindungsgemäßen Verfahrens ist dadurch gekennzeichnet, dass der Ionenaustauscher auf einem Polystyrolgerüst mit Sulfonsäuregruppen oder auf einem perfluorierten lonenaustauscherharz mit Sulfonsäuregruppen basiert.

Die kommerziell verfügbaren sauren Kationenaustauscher sind unter den Handelsnamen Lewatit^{®} (Lanxess), Purolite^{®} (The Purolite Company), Dowex^{®} (Dow Chemical Company), Amberlite^{®} (Rohm and Haas Company), Amberlyst^{™} (Rohm and Haas Company) bekannt. Als erfindungsgemäß bevorzugte saure Kationenaustauscher seien beispielsweise genannt: Lewatit^{®} K 1221, Lewatit^{®} K 1461, Lewatit^{®} K 2431, Lewatit^{®} K 2620, Lewatit^{®} K 2621, Lewatit^{®} K 2629, Lewatit^{®} K 2649, Amberlite^{®} IR 120, Amberlyst^{™} 131, Amberlyst^{™} 15, Amberlyst^{™} 31, Amberlyst^{™} 35, Amberlyst^{™} 36, Amberlyst^{™} 39, Amberlyst^{™} 46, Amberlyst^{™} 70, Purolite^{®} SGC650, Purolite^{®} C100H, Purolite^{®} C150H, Dowex^{®} 50X8, Dowex^{®} 88, Serdolit^{®} rot und Nation^{®} NR-50.

Im Rahmen einer bevorzugten Ausführungsform führt man die erfindungsgemäß durchzuführende Umsetzung von Verbindung (II) in Gegenwart mindestens eines sauren Kationenaustauschers durch, der ausgewählt ist aus der Gruppe der Kationenaustauscher umfassend Lewatit^{®} K 1221, Lewatit^{®} K 2629, Amberlyst^{™} 131, Amberlyst^{™} 35 Purolite^{®} SGC650, Purolite^{®} C100H, Purolite^{®} C15OH, Amberlite^{®} IR 120, Dowex^{®} 88, und Dowex^{®} 50X8.

Erfindungsgemäß insbesondere bevorzugte saure Kationentauscher sind die Kationenaustauscher Amberlyst^{™} 35, Dowex^{®} 88, und/oder Amberlite^{®} IR 120.

Ein erfindungsgemäß ganz besonders bevorzugter saurer Kationenaustauscher ist Amberlyst^{™} 35, der wie die anderen genannten Kationenaustauscher kommerziell verfügbar ist.

Die sauren lonentauscherharze werden in der Regel mit Salzsäure und/oder Schwefelsäure regeneriert.

Die Hydrierung im Schritt b) kann auf an sich herkömmliche Weise mit einem Hydrierkatalysator des Stands der Technik ausgeführt werden. Die Hydrierung kann katalytisch entweder in der Gas- oder Flüssigphase erfolgen. Vorzugweise wird die Hydrierung im Schritt b) in flüssiger Phase in Gegenwart eines heterogegen Hydrierkatalysators und eines wasserstoffhaltigen Gases durchgeführt.

Als Hydrierkatalysatoren kommen prinzipiell alle zur Hydrierung von ungesättigten organischen Verbindungen geeigneten homogenen und heterogenen Katalysatoren in Betracht. Dazu zählen z. B. Metalle, Metalloxide, davon verschiedene Metallverbindungen oder Gemische davon. Geeignete Hydrierkatalysatoren enthalten vorzugsweise wenigstens ein Übergangsmetall, bevorzugt aus den Nebengruppen I. und VI. bis VIII. des Periodensystems der Elemente. Dazu zählen vorzugsweise Pd, Pt, Ni, Rh, Ru, Co, Fe, Zn, Cu, Re oder deren Mischungen.

Der Hydrierkatalysator kann wenigstens ein weiteres Metall/Element enthalten, das von den zuvor beschriebenen Metallen verschieden ist. Das weitere Metall/Element ist vorzugsweise ausgewählt unter Alkalimetallen, Erdalkalimetalle, Aluminium, Silizium, Lanthanoide und Mischungen davon.

Der Anteil des weiteren Metalls/Element ist vorzugsweise im Bereich von 0,1 bis 10 Gew.-% bezogen auf das Gesamtgewicht des aktiven Teil des Hydrierkatalysators (exklusiv Träger).

Die Katalysatoren können allein aus den Aktivkomponenten bestehen, oder die Aktivkomponenten können auf Trägern aufgebracht sein. Als Trägermaterialien eignen sich z. B. Zirkoniumdioxid, Bariumoxid, Zinkoxid, Magnesiumoxid, Titanoxid, Aluminumoxid, TiO₂-Al₂O₃, ZrO₂-Al₂O₃, Zeolithen, Hydrotalcit, Siliciumcarbid, Wolframcarbid, Siliciumdioxid, Kohlenstoff, speziell Aktivkohle oder sulfatierter Kohlenstoff, Diatomeenerde, Tonerde, Bariumsulfat, Calciumcarbonat und Mischungen.

In einer Ausführungsform umfassen die Trägermaterialen gleichzeitig eine erfindungsgemäß eingesetzte Säure oder bestehen daraus.

Zur Erhöhung der katalytischen Aktivität können Ni, Cu oder Co, auch in Form der Raney-Katalysatoren, Pd, Pt, Rh, Ru, Co, Fe, Zn, Cu, oder deren Mischungen als Metallschwamm mit einer sehr großen Oberfläche verwendet werden.

Bevorzugt wird für die Hydrierung im Schritt b) des erfindungsgemäßen Verfahrens vorteilhaft Palladium auf Kohlenstoff, Palladium auf Al₂O₃, Palladium auf SiO₂ oder Platin auf Kohlenstoff eingesetzt. Besonders bevorzugt wird vorteilhaft Palladium auf Kohlenstoff eingesetzt.

Andere geeignete Katalysatoren enthalten z. B. 80 bis 100 Gew.-% Nickel und/oder Cobalt und bis zu 20 Gew.-% aktivierende Metalle wie Kupfer und/oder Chrom. Besonders vorteilhaft werden solche Katalysatoren als Trägerkatalysatoren verwendet.

Der Gehalt an katalytisch aktiven Metallen solcher Trägerkatalysatoren, wobei das Trägermaterial Kohlenstoff ist, beträgt in der Regel 0,05 bis 10 Gew.-%, bezogen auf die Summe von katalytisch aktiven Metallen und Träger.

Der Gehalt an katalytisch aktiven Metallen solcher Trägerkatalysatoren, wobei das Trägermaterial ein Oxid, z. B. Al₂O₃ oder SiO₂ ist, beträgt in der Regel 0,01 bis 1 Gew.-%, bezogen auf die Summe von katalytisch aktiven Metallen und Träger.

Die Katalysatoren können zur Hydrierung in Schritt b) als Formkörper eingesetzt werden. Beispiele umfassen Katalysatorextrudate, wie Stränge, Rippstränge und andere Extrudatformen, Schalenkatalysatoren, Tabletten, Ringe, Kugeln, Splitt, etc.

Bevorzugt wird die Hydrierung in Schritt b) bei einer Temperatur von 60 bis 200 ° C, vorzugsweise 120 bis 150 ° C, insbesondere 135 bis 145 ° C, durchgeführt.

Sofern man die Umsetzung in der Gasphase durchführt, liegt der Druck vorzugsweise in einem Bereich von 0,9 bis 50 bar, besonders bevorzugt 1 bis 20 bar.

Sofern man die Umsetzung in der Flüssigphase durchführt, liegt der Druck vorzugsweise in einem Bereich von 0,9 bis 200 bar, insbesondere von 40 bis 80 bar.

Die Hydrierung in Schritt b) kann in einem Reaktor oder mehreren hintereinander geschalteten Reaktoren durchgeführt werden. Die Hydrierung kann diskontinuierlich oder kontinuierlich erfolgen. Zur diskontinuierlichen Hydrierung kann z. B. ein Druckgefäß eingesetzt werden. Geeignete Druckgefäße sind z. B. Autoklaven, die mit einer Vorrichtung zum Beheizen und zum Rühren des Reaktorinhalts ausgestattet sind. Bevorzugt erfolgt die Hydrierung in der Flüssigphase über einem Festbett, vorzugsweise in Sumpf- oder Rieselfahrweise oder in Form einer Suspensionskatalyse. Die Festbettfahrweise kann dabei z. B. in Sumpf- oder in Rieselfahrweise durchgeführt werden. Dabei werden die Katalysatoren vorzugsweise als Formkörper eingesetzt, z. B. in Form von gepressten Zylindern, Tabletten, Pastillen, Wagenrädern, Ringen, Sternen oder Strangpresslingen, wie Vollsträngen, polylobären Strängen, Hohlsträngen, Wabenkörpern, etc.

Bei der Suspensionsfahrweise werden ebenfalls heterogene Katalysatoren eingesetzt. Die heterogenen Katalysatoren werden dabei zumeist in feinverteiltem Zustand eingesetzt und liegen im Reaktionsmedium feinteilig suspendiert vor.

Bei der Hydrierung an einem Festbett wird ein Reaktor eingesetzt, in dessen Innenraum ein Festbett angeordnet ist, durch das das Reaktionsmedium strömt. Das Festbett kann dabei aus einer einzigen oder aus mehreren Schüttungen gebildet sein. Jede Schüttung kann dabei eine oder mehrere Zonen aufweisen, wobei wenigstens eine der Zonen ein als Hydrierkatalysator aktives Material enthält. Jede Zone kann dabei ein oder mehrere verschiedene katalytisch aktive Materialien aufweisen und/oder ein oder mehrere verschiedene inerte Materialien aufweisen.

Verschiedene Zonen können jeweils gleiche oder verschiedene Zusammensetzungen aufweisen. Es ist auch möglich, mehrere katalytisch aktive Zonen vorzusehen, die beispielsweise durch inerte Schüttungen voneinander getrennt sind. Die einzelnen Zonen können auch unterschiedliche katalytische Aktivität aufweisen. Dazu können verschiedene katalytisch aktive Materialien eingesetzt werden und/oder wenigstens einer der Zonen ein inertes Material beigemischt werden. Das Reaktionsmedium, das durch das Festbett strömt, enthält erfindungsgemäß mindestens eine flüssige Phase. Das Reaktionsmedium kann auch zusätzlich eine gasförmige Phase enthalten.

Als Reaktoren bei der Hydrierung in Suspension kommen insbesondere Schlaufenapparate, wie Strahlschlaufen oder Propellerschlaufen, Rührkessel, die auch als Rührkesselkaskaden ausgestaltet sein können, Blasensäulen oder Air-Lift-Reaktoren zum Einsatz.

Vorzugsweise erfolgt die Hydrierung in Schritt b) in der Suspensionsfahrweise.

Die Hydrierung kann ohne oder mit Zusatz eines Lösungsmittels erfolgen. Als Lösungsmittel kommen Alkohole, Ether, Kohlenwasserstoffe, wie beispielsweise Methanol, Ethanol, Isopropanol, Dioxan, Tetrahydrofuran, n-Pentan, Hexan, Cyclohexan, Toluol, etc., in Frage. Bevorzugt erfolgt die Hydrierung in Schritt b) ohne Zusatz eines Lösungsmittels.

Zur Hydrierung in Schritt b) kann man die in Schritt a) erhaltene Verbindung der Formel (II) mit einem wasserstoffhaltigen Gas und einem Hydrierkatalysator in Kontakt bringen. Geeignete wasserstoffhaltige Gase sind ausgewählt unter Wasserstoff und Gemischen von Wasserstoff mit wenigstens einem inerten Gas. Geeignete inerte Gase sind z. B. Stickstoff oder Argon. Bevorzugt wird zur Hydrierung in Schritt b) Wasserstoff in unverdünnter Form, üblicherweise in einer Reinheit von etwa 99,9 Vol.-%, eingesetzt.

Durch die Hydrierung in Schritt b) werden die Verbindungen der Formel (II) in 2-substituierte 4-Methyl-tetrahydropyrane (I) überführt. Bevorzugt enthält das zur Hydrierung eingesetzte Ausgangsmaterial Verbindungen der Formel (II), wobei der Rest R¹ die oben genannten Bedeutungen aufweist. R¹ steht bevorzugt für Isobutyl.

In einer speziellen Ausführungsform wird durch die Hydrierung in Schritt b) die Verbindungen (II) in 2-Isobutyl-4-methyl-tetrahydropyran (la) (Dihydrorosenoxid) überführt.

Die in Schritt b) erhaltene Verbindung der Formel (I) weist vorzugsweise ein Diastereomerenverhältnis des cis-Diastereomeren zum trans-Diastereomeren in einem Bereich von 10 : 90 bis 90 : 10, besonders bevorzugt von 65 : 35 bis 90 : 10, auf.

Die in Schritt b) erhaltene Verbindung der Formel (I) lässt sich durch einfache Reinigungsschritte in eine zur kommerziellen Verwendung geeignete Form überführen.

Falls gewünscht, kann die in Schritt b) erhaltene Verbindung der Formel (I) einer weiteren Aufarbeitung unterzogen werden. Dazu kann die in Schritt b) erhaltene Verbindung (I) prinzipiell üblichen, dem Fachmann bekannten Reinigungsverfahren unterzogen werden. Dazu zählt beispielsweise eine Filtration, eine Neutralisation, eine Destillation, eine Extraktion oder eine Kombination davon.

Bevorzugt wird aus dem in Schritt b) erhaltenen Hydrierprodukt eine an 2-substituierten 4-Methyl-tetrahydropyranen (I) angereicherte Fraktion und eine an 2-substituierten 4-Methyl-tetrahydropyranen (I) abgereicherte Fraktion isoliert.

Bevorzugt wird die in Schritt b) erhaltene Verbindung (I) einer destillativen Auftrennung unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Bevorzugt wird die in Schritt b) erhaltene Verbindung (I) in Schritt c) einer destillativen Auftrennung in wenigstens einer Destillationskolonne unterzogen, die mit trennwirksamen Einbauten versehen ist.

Bevorzugt wird in Schritt c) aus der in Schritt b) erhaltenen Verbindung (I) eine an 2-substituierten 4-Methyl-tetrahydropyranen (I) angereicherte Fraktion isoliert, wobei das Diastereomerenverhältnis des cis-Diastereomeren zum trans-Diastereomeren in einem Bereich von 10 : 90 bis 90 : 10, bevorzugt von 65 : 35 bis 90 : 10, liegt.

Zur Entfernung von weiteren wasserlöslichen Verunreinigungen kann die in Schritt c) erhaltene an 2-substituierten 4-Methyl-tetrahydropyranen (I) angereicherte Fraktion wenigstens einem Waschschritt mit Wasser unterzogen werden. Alternativ oder zusätzlich kann die in Schritt c) erhaltene an 2-substituierten 4-Methyl-tetrahydropyranen (I) angereicherte Fraktion einer weiteren destillativen Aufreinigung unterzogen werden.

Die folgenden Beispiele dienen der Verdeutlichung der Erfindung.

### BEISPIELE

Gaschromatographische Analysen wurden nach der folgenden Methode durchgeführt:
Säule: DB WAX 30 m x 0,32 mm;
FD 0,25 µm;
Injektortemperatur: 200 ° C; Detektortemperatur 280 ° C;
Temperaturprogramm: Anfangstemp.: 50 ° C, mit 3 ° C/min auf 170 ° C, mit 20 ° C/min auf 230 ° C, 7 Min isotherm;
Retentionszeiten: 2-Isobutyl-4-methyl-tetrahydropyran-4-ol t_{R} = 28,9 min und 30,4
   cis-Dihydrorosenoxid t_{R} = 8,77 min
   trans-Dihydrorosenoxid t_{R} = 10,09 min

Konzentrationen der erhaltenen Rohprodukte (Gew.-%) wurden GC-analytisch mit internem Standard ermittelt.

### 1. Herstellung von 2-Isobutyl-4-methyl-tetrahydropyran ausgehend von 2-Isobutyl-4-methyl-tetrahydropyran-4-ol in Methanol

12 g von 2-Isobutyl-4-methyl-tetrahydropyran-4-ol (Isomerenverhältnis 24 : 76), 28 g Methanol, 0,2 g Katalysator Pd/C (10 % Pd auf C) und 0,2 g Amberlyst 35 dry werden in einem Autoklaven eingewogen. Dieser wird verschlossen und je einmal mit Stickstoff und Wasserstoff gespült. Anschließend werden zunächst 30 bar Wasserstoff aufgepresst, dann auf 140 ° C erhitzt und nach Erreichen der Reaktionstemperatur der Druck auf 50 bar eingeregelt. Der Versuch wird unter diesen Bedingungen für 12 Stunden gerührt, nach 1 h, 3 h und 5 h wird jeweils Wasserstoff nachgepresst, um den Druck auf 50 bar zu halten. Anschließend wird der Autoklav entspannt und abgekühlt. Katalysator und Ionentauscher werden abfiltriert, die resultierende Lösung ist farblos und klar.

Bei einem 2-Isobutyl-4-methyl-tetrahydropyran-4-ol-Umsatz von >99 % wurde mit einer Selektivität von 86 % bezüglich 2-Isobutyl-4-methyl-tetrahydropyran-4-ol 2-Isobutyl-4-methyl-tetrahydropyran gebildet. Das cis/trans-Verhältnis beträgt 5,33 : 1.

### 2. Herstellung von 2-Isobutyl-4-methyl-tetrahydropyran ausgehend von 2-Isobutyl-4-methyl-tetrahydropyran-4-ol lösungsmittelfrei

36 g 2-Isobutyl-4-methyl-tetrahydropyran-4-ol (Isomerenverhältnis 24 : 76), 0,4 g Katalysator Pd/C (10 % Pd auf C) und 0,4 g Amberlyst 35 dry werden in einem Autoklaven eingewogen. Dieser wird verschlossen und je einmal mit Stickstoff und Wasserstoff gespült. Anschließend werden zunächst 30 bar Wasserstoff aufgepresst, dann auf 140 ° C erhitzt und nach Erreichen der Reaktionstemperatur der Druck auf 50 bar eingeregelt. Der Versuch wird unter diesen Bedingungen für 12 Stunden gerührt, nach 1 h, 3 h und 5 h wird jeweils Wasserstoff nachgepresst, um den Druck auf 50 bar zu halten. Anschließend wird der Autoklav entspannt und abgekühlt. Katalysator und Ionentauscher werden abfiltriert, die resultierende Lösung ist farblos und klar.

Bei einem 2-Isobutyl-4-methyl-tetrahydropyran-4-ol-Umsatz von 60 % wurde mit einer Selektivität von 50 % bezüglich 2-Isobutyl-4-methyl-tetrahydropyran-4-ol 2-Isobutyl-4-methyl-tetrahydropyran gebildet. Das cis/trans-Verhältnis beträgt 6,1 : 1.

### 3. Herstellung von 2-Isobutyl-4-methyl-tetrahydropyran ausgehend von 2-Isobutyl-4-methyl-tetrahydropyran-4-ol in Methanol

12 g von 2-Isobutyl-4-methyl-tetrahydropyran-4-ol (Isomerenverhältnis 24 : 76), 28 g Methanol, 0,4 g Katalysator Pd/C (5 % Pd auf C) und 0,4 g Amberlyst 35 dry werden in einem Autoklaven eingewogen. Dieser wird verschlossen und je einmal mit Stickstoff und Wasserstoff gespült. Anschließend werden zunächst 30 bar Wasserstoff aufgepresst, dann auf 140 ° C erhitzt und nach Erreichen der Reaktionstemperatur der Druck auf 50 bar eingeregelt. Der Versuch wird unter diesen Bedingungen für 12 Stunden gerührt, nach 1 h, 3 h und 5 h wird jeweils Wasserstoff nachgepresst, um den Druck auf 50 bar zu halten. Anschließend wird der Autoklav entspannt und abgekühlt. Katalysator und Ionentauscher werden abfiltriert, die resultierende Lösung ist farblos und klar.

Bei einem 2-Isobutyl-4-methyl-tetrahydropyran-4-ol-Umsatz von >81,2 % wurde mit einer Selektivität von 65,6 % bezüglich 2-Isobutyl-4-methyl-tetrahydropyran-4-ol 2-Isobutyl-4-methyl-tetrahydropyran gebildet. Das cis/trans-Verhältnis beträgt 6,06 : 1.

### 4. Herstellung von 2-Isobutyl-4-methyl-tetrahydropyran ausgehend von 2-Isobutyl-4-methyl-tetrahydropyran-4-ol in Methanol

12 g von 2-Isobutyl-4-methyl-tetrahydropyran-4-ol (Isomerenverhältnis 24 : 76), 28 g Methanol, 0,7 g Katalysator Pd/C (5 % Pd auf C) und 0,7 g Amberlyst 35 dry werden in einem Autoklaven eingewogen. Dieser wird verschlossen und je einmal mit Stickstoff und Wasserstoff gespült. Anschließend werden zunächst 30 bar Wasserstoff aufgepresst, dann auf 120 ° C erhitzt und nach Erreichen der Reaktionstemperatur der Druck auf 80 bar eingeregelt. Der Versuch wird unter diesen Bedingungen für 12 Stunden gerührt, nach 1 h, 3 h und 5 h wird jeweils Wasserstoff nachgepresst, um den Druck auf 50 bar zu halten. Anschließend wird der Autoklav entspannt und abgekühlt. Katalysator und Ionentauscher werden abfiltriert, die resultierende Lösung ist farblos und klar.

Bei einem 2-Isobutyl-4-methyl-tetrahydropyran-4-ol-Umsatz von 56,7 % wurde mit einer Selektivität von 62 % bezüglich 2-Isobutyl-4-methyl-tetrahydropyran-4-ol 2-Isobutyl-4-methyl-tetrahydropyran gebildet. Das cis/trans-Verhältnis beträgt 5,17 : 1.

## Patentansprüche

1. Verfahren zur Herstellung von Verbindungen der allgemeinen Formel (I) 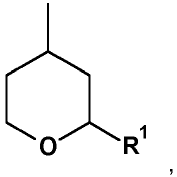 wobei R¹ ausgewählt ist unter
geradkettigem oder verzweigtem C₁-C₁₂-Alkyl, wobei Alkyl unsubstituiert oder mit wenigstens einen Substituenten, ausgewählt unter Aryl, C₁-C₁₂-Alkoxy und C₁-C₁₂-alkylcarbonyl aufweist,
unsubstituiertem oder mit 1, 2, 3 oder 4 Substituenten, ausgewählt unter C₁-C₁₂-Alkyl, C₁-C₁₂-Alkoxy, C₁-C₁₂-Alkyl, C₁-C₁₂-Alkoxy, Phenyl und Benzyl, substituiertem Cycloalkyl mit insgesamt 3 bis 20 Kohlenstoffatomen,
umfassend die Schritte:
a) Bereitstellung wenigstens einer Verbindung der allgemeinen Formel (II) 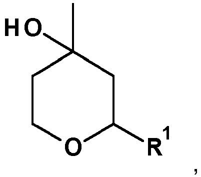 wobei R¹ die zuvor angegebene Bedeutung hat,
b) Hydrierung der Verbindung (II) in Gegenwart eines Hydrierkatalysators unter sauren Bedingungen,
wobei es sich um eine Eintopfsynthese handelt.

2. Verfahren nach Anspruch 1, wobei R¹ ein geradkettiges oder verzweigtes C₁-C₆-Alkyl ist, wobei Alkyl unsubstituiert oder wenigstens einen Substituenten aufweist, der ausgewählt ist unter Phenyl und C₁-C₆-Alkoxy.

3. Verfahren nach einem der vorhergehenden Ansprüche, wobei R¹ für Methyl, Ethyl, n-Propyl, Isopropyl, n-Butyl, Isobutyl, n-Pentyl, n-Hexyl oder Phenyl, bevorzugt für Isobutyl, steht.

4. Verfahren nach einem der vorhergehenden Ansprüche, wobei das IsomerenVerhältnis von cis : trans von Verbindung (I) im Bereich von 10 : 90 bis 90 : 10 liegt, bevorzugt im Bereich von 65 : 35 bis 90 : 10 liegt.

5. Verfahren nach einem der vorhergehenden Ansprüche, wobei die Hydrierung in Schritt b) in Gegenwart einer Säure, ausgewählt unter wenigstens einer Protonensäure, wenigstens einer Lewissäure, wenigstens einem sauren Ionenaustauscher, wenigstens einem oxidischen sauren Festkörper, wenigstens einer sauren molekularen Elementverbindung und Gemischen davon, stattfindet.

6. Verfahren nach einem der Ansprüche 1 bis 5, wobei die Hydrierung in Schritt b) in Gegenwart einer Säure erfolgt, die ausgewählt ist unter Salzsäure, Schwefelsäure, Phosphorsäure, Salpetersäure, Essigsäure, Ameisensäure, Trifluormethylsulfonsäure, Methansulfonsäure, p-Toluolsulfonsäure, Aluminiumchlorid, Bortrifluorid, Zinkchlorid, Phosphorpentafluorid, Arsentrifluorid, Zinntetrachlorid, Titantetrachlorid, Antimonpentafluorid und Gemischen davon.

7. Verfahren nach einem der Ansprüche 1 bis 5, wobei die Hydrierung in Schritt b) in Gegenwart eines sauren Kationenaustauschers durchgeführt wird.

8. Verfahren nach einem der Ansprüche 1 bis 5, wobei die Hydrierung in Schritt b) in Gegenwart eines oxidischen sauren Festkörper durchgeführt wird, der ausgewählt ist unter Zeolithen, Silikaten, Aluminaten, Alumosilikaten und Tonen.

9. Verfahren nach einem der vorhergehenden Ansprüche, wobei der Katalysator wenigstens ein Übergangsmetall umfasst, das ausgewählt ist unter Pd, Pt, Ni, Rh, Ru, Co, Fe, Zn, Cu, Re oder Gemischen davon.

10. Verfahren nach einem der vorhergehenden Ansprüche, wobei es sich bei dem Hydrierungskatalysator um einen geträgerten Katalysator handelt.

11. Verfahren nach einem der vorhergehenden Ansprüche, wobei der Träger des Katalysators ausgewählt ist unter Zirkoniumdioxid, Zinkoxid, Magnesiumoxid, Titanoxid, Aluminumoxid, Bariumoxid, TiO₂-Al₂O₃, ZrO₂-Al₂O₃, Zeolithen, Hydrotalcit, Siliciumcarbid, Wolframcarbid, Siliciumdioxid, Kohlenstoff, speziell Aktivkohle oder sulfatiertem Kohlenstoff, Diatomeenerde, Tonerde, Bariumsulfat, Calciumcarbonat und Mischungen davon.

12. Verfahren nach einem der vorhergehenden Ansprüche, wobei die Temperatur in Schritt b) im Bereich von 60 bis 200 °C, bevorzugt im Bereich von 120 bis 150 °C, liegt.

13. Verfahren nach einem der vorhergehenden Ansprüche, wobei der Druck in Schritt b) in einem Bereich von 900 mbar bis 200 bar, bevorzugt 40 bis 80 bar, liegt.

## Claims

1. A method for preparing compounds of the general formula (I) 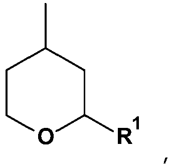
where R¹ is selected from
straight-chain or branched C₁-C₁₂-alkyl, where alkyl is unsubstituted or has at least one substituent selected from aryl, C₁-C₁₂-alkoxy and C₁-C₁₂-alkylcarbonyl,
cycloalkyl having a total of 3 to 20 carbon atoms that is unsubstituted or substituted by 1, 2, 3 or 4 substituents selected from C₁-C₁₂-alkyl, C₁-C₁₂-alkoxy, C₁-C₁₂-alkyl, C₁-C₁₂-alkoxy, phenyl and benzyl,
comprising the steps of:
a) providing at least one compound of the general formula (II) 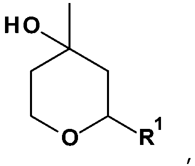 where R¹ is as defined above,
b) hydrogenating the compound (II) in the presence of a hydrogenation catalyst under acidic conditions,
wherein it is a one-pot synthesis.

2. The method according to claim 1, wherein R¹ is a straight-chain or branched C₁-C₆-alkyl, where alkyl is unsubstituted or has at least one substituent selected from phenyl and C₁-C₆-alkoxy.

3. The method according to either of the preceding claims, wherein R¹ is methyl, ethyl, n-propyl, isopropyl, n-butyl, isobutyl, n-pentyl, n-hexyl or phenyl, preferably isobutyl.

4. The method according to any of the preceding claims, wherein the isomeric ratio of cis : trans of compound (I) is in the range from 10 : 90 to 90 : 10, preferably in the range from 65 : 35 to 90 : 10.

5. The method according to any of the preceding claims, wherein the hydrogenation in step b) is carried out in the presence of an acid selected from at least one protic acid, at least one Lewis acid, at least one acidic ion exchanger, at least one oxidic acidic solid, at least one acidic molecular element compound and mixtures thereof.

6. The method according to any of claims 1 to 5, wherein the hydrogenation in step b) is carried out in the presence of an acid which is selected from hydrochloric acid, sulfuric acid, phosphoric acid, nitric acid, acetic acid, formic acid, trifluoromethylsulfonic acid, methanesulfonic acid, p-toluenesulfonic acid, aluminum chloride, boron trifluoride, zinc chloride, phosphorus pentafluoride, arsenic trifluoride, tin tetrachloride, titanium tetrachloride, antimony pentafluoride and mixtures thereof.

7. The method according to any of claims 1 to 5, wherein the hydrogenation in step b) is carried out in the presence of an acidic cation exchanger.

8. The method according to any of claims 1 to 5, wherein the hydrogenation in step b) is carried out in the presence of an oxidic acidic solid which is selected from zeolites, silicates, aluminates, aluminosilicates and clays.

9. The method according to any of the preceding claims, wherein the catalyst comprises at least one transition metal selected from Pd, Pt, Ni, Rh, Ru, Co, Fe, Zn, Cu, Re or mixtures thereof.

10. The method according to any of the preceding claims, wherein the hydrogenation catalyst is a supported catalyst.

11. The method according to any of the preceding claims, wherein the catalyst support is selected from zirconium dioxide, zinc oxide, magnesium oxide, titanium oxide, aluminum oxide, barium oxide, TiO₂-Al₂O₃, ZrO₂-Al₂O₃, zeolites, hydrotalcite, silicon carbide, tungsten carbide, silicon dioxide, carbon, especially activated carbon or sulfated carbon, diatomaceous earth, clay, barium sulfate, calcium carbonate and mixtures thereof.

12. The method according to any of the preceding claims, wherein the temperature in step b) is in the range from 60 to 200°C, preferably in the range from 120 to 150°C.

13. The method according to any of the preceding claims, wherein the pressure in step b) is in a range from 900 mbar to 200 bar, preferably 40 to 80 bar.

## Revendications

1. Procédé de préparation de composés de formule générale (I) 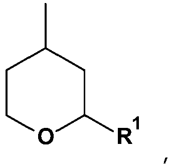 dans laquelle R¹ est choisi parmi
C₁-C₁₂-alkyle linéaire ou ramifié, alkyle étant non substitué ou comprenant au moins un substituant, choisi parmi aryle, C₁-C₁₂-alcoxy et C₁-C₁₂-alkylcarbonyle,
cycloalkyle, comprenant au total 3 à 20 atomes de carbone, non substitué ou substitué par 1, 2, 3 ou 4 substituants, choisis parmi C₁-C₁₂-alkyle, C₁-C₁₂-alcoxy, C₁-C₁₂-alkyle, C₁-C₁₂-alcoxy, phényle et benzyle,
comprenant les étapes :
a) mise à disposition d'au moins un composé de formule générale (II) 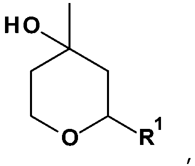 dans laquelle R¹ présente la signification indiquée ci-dessus,
b) hydrogénation du composé (II) en présence d'un catalyseur d'hydrogénation dans des conditions acides ;
la synthèse étant une synthèse monotope.

2. Procédé selon la revendication 1, R¹ représentant un C₁-C₆-alkyle linéaire ou ramifié, alkyle étant non substitué ou présentant au moins un substituant qui est choisi parmi phényle et C₁-C₆-alcoxy.

3. Procédé selon l'une des revendications précédentes, R¹ représentant méthyle, éthyle, n-propyle, isopropyle, n-butyle, isobutyle, n-pentyle, n-hexyle ou phényle, de préférence isobutyle.

4. Procédé selon l'une des revendications précédentes, le rapport d'isomères cis:trans du composé (I) se situant dans la plage de 10:90 à 90:10, de préférence dans la plage de 65:35 à 90:10.

5. Procédé selon l'une des revendications précédentes, l'hydrogénation dans l'étape b) ayant lieu en présence d'un acide choisi parmi au moins un acide protonique, au moins un acide de Lewis, au moins un échangeur ionique acide, au moins un solide acide oxyde, au moins un composé moléculaire acide et leurs mélanges.

6. Procédé selon l'une des revendications 1 à 5, l'hydrogénation dans l'étape b) étant effectuée en présence d'un acide choisi parmi l'acide chlorhydrique, l'acide sulfurique, l'acide phosphorique, l'acide nitrique, l'acide acétique, l'acide formique, l'acide trifluorométhylsulfonique, l'acide méthanesulfonique, l'acide p-toluènesulfonique, le chlorure d'aluminium, le trifluorure de bore, le chlorure de zinc, le pentafluorure de phosphore, le trifluorure d'arsenic, le tétrachlorure d'étain, le tétrachlorure de titane, le pentafluorure d'antimoine et leurs mélanges.

7. Procédé selon l'une des revendications 1 à 5, l'hydrogénation dans l'étape b) étant réalisée en présence d'un échangeur cationique acide.

8. Procédé selon l'une des revendications 1 à 5, l'hydrogénation dans l'étape b) étant réalisée en présence d'un solide acide oxyde choisi parmi les zéolites, les silicates, les aluminates, les aluminosilicates et les argiles.

9. Procédé selon l'une des revendications précédentes, le catalyseur comprenant au moins un métal de transition choisi parmi Pd, Pt, Ni, Rh, Ru, Co, Fe, Zn, Cu, Re ou leurs mélanges.

10. Procédé selon l'une des revendications précédentes, le catalyseur d'hydrogénation étant un catalyseur supporté.

11. Procédé selon l'une des revendications précédentes, le support du catalyseur étant choisi parmi le dioxyde de zirconium, **l'oxyde** de zinc, **l'oxyde** de magnésium, l'oxyde de titane, l'oxyde d'aluminium, l'oxyde de baryum, le TiOᵣAl₂O₃, le ZrO₂-Al₂O₃, les zéolithes, l'hydrotalcite, le carbure de silicium, le carbure de tungstène, le dioxyde de silicium, le carbone, spécifiquement le charbon actif ou sulfaté, les terres de diatomées, l'alumine, le sulfate de baryum, le carbonate de calcium et leurs mélanges.

12. Procédé selon l'une des revendications précédentes, la température dans l'étape b) étant située dans la plage de 60 à 200 °C, de préférence dans la plage de 120 à 150 °C.

13. Procédé selon l'une des revendications précédentes, la pression dans l'étape b) étant située dans une plage de 900 mbars à 200 bars, de préférence de 40 à 80 bars.
